# EUROPÄISCHE PATENTANMELDUNG

(11) **EP 0 705 605 A2**
(43) Veröffentlichungstag der Anmeldung: **10.04.1996**
(21) Anmeldenummer: 95114509.3
(22) Anmeldetag: 15.09.1995
(51) Int. Cl.: A61K 31/22, A61K 31/23

(54) **Verwendung von Fettsäureestern zur Bekämpfung von Superinfektionen**

(30) Priorität: 29.09.1994 DE 4434781
(71) Anmelder: Beiersdorf Aktiengesellschaft, D-20245 Hamburg (DE)
(72) Erfinder: Wolf, Florian, Dr., D-20251 Hamburg (DE); Kahlert, Michael, D-22041 Hamburg (DE)

(57) **Zusammenfassung**

Verwendung eines oder mehrerer Fettsäureester ein- und/oder mehrwertiger Alkohole als Wirkprinzip gegen Superinfektionen.

## Beschreibung

Die vorliegende Erfindung betrifft Wirkstoffe und dermatologische Zubereitungen, solche Wirkstoffe enthaltend, gegen Superinfektionen, insbesondere Superinfektionen der Haut.

Die gesunde menschliche Haut ist mit einer Vielzahl nichtpathogener Mikroorganismen besiedelt. Diese sogenannte Mikroflora der Haut ist nicht nur unschädlich, sie stellt auch einen wichtigen Schutz zur Abwehr opportunistischer oder pathogener Keime dar.

Bei bestehendem Primärinfekt, d.h., der normalen Keimbesiedelung der Haut, eintretende Neuinfektion mit hohen Keimzahlen eines oder mehrerer im wesentlichen identischer oder zumindest artverwandter Erreger, beispielsweise Staphylokokken, kann bei Zusammentreffen ungünstiger Einflüssen eine "Superinfektion" der befallenen Haut auftreten. Die normale Mikroflora der Haut (oder eines anderen Körperorgans) wird dabei von dem Sekundärerreger regelrecht überwuchert.

Solche Superinfektionen können sich, in Abhängigkeit vom betreffenden Keim, in günstig verlaufenden Fällen in unangenehmen Hauterscheinungen (Juckreiz, unschönes äußeres Erscheinungsbild) äußern. In ungünstig verlaufenden Fällen können sie aber zu großflächiger Zerstörung der Haut führen, im schlimmsten Falle sogar im Tode des Patienten gipfeln.

Superinfektionen der vorab geschilderten Art sind z.B. beim Vollbild von AIDS häufig auftretende Sekundärerkrankungen.

An sich unschädliche, aber unter Umständen auch ausgesprochen pathogene Keime überwuchern auf diese Weise die gesunde Hautflora. Bei AIDS allerdings sind auch andere Körperorgane von Superinfektionen betroffen.

Zwar ist es im Einzelfalle ohne weiteres möglich, Superinfektionen mit Antibiotika zu bekämpfen, meistens haben solche Substanzen aber den Nachteil unangenehmer Nebenwirkungen. Oft sind Patienten beispielsweise gegen Penicilline allergisch, weswegen eine entsprechende Behandlung sich in einem solchen Falle verbieten würde.

Ferner haben topisch verabreichte Antibiotika den Nachteil, daß sie die Hautflora nicht nur vom Sekundärerreger befreien, sondern auch die an sich physiologische Hautflora stark beeinträchtigen und der natürliche Heilungsprozeß auf diese Weise wieder gebremst wird.

Aufgabe der vorliegenden Aufgabe war, die Nachteile des Standes der Technik zu beseitigen und Substanzen und Zubereitungen, solche Substanzen enthaltend, zur Verfügung zu stellen, durch deren Verwendung Superinfektionen geheilt werden können, wobei die physiologische Hautflora keine nennenswerte Einbußen erleidet.

Es wurde gefunden, und darin liegt die Lösung all dieser Aufgaben, daß die Verwendung eines oder mehrerer Fettsäureester ein- und/oder mehrwertiger Alkohole als Wirkprinzip gegen Superinfektionen den Nachteilen des Standes der Technik abhilft.

Es hat sich in erstaunlicher Weise herausgestellt, daß die erfindungsgemäßen Verbindungen Superinfektionen beseitigen und das natürliche Gleichgewicht der Hautflora wieder herstellen.

Die erfindungsgemäßen Wirkstoffkombinationen sind hervorragend wirksam gegen Superinfektionen, insbesondere gegen Staphylokokken, Pityrosporum ovale sowie Dermatophyten.

Zwar beschreibt die DE-OS 42 37 367 die antimykotische Verwendung der erfindungsgemäßen Fettsäureester. Der Stand der Technik konnte jedoch keinen Hinweis auf die erfindungsgemäße Wirkung gegen Superinfektionen geben.

Fettsäurester im Sinne der vorliegenden Erfindung sind Ester der allgemeinen Formel 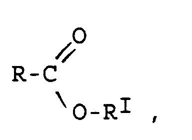
welche formal Veresterervngsprodukte einer Fettsäure der allgemeinen Formel 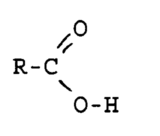
sowie eines ein- oder mehrwertigen Alkohols der allgemeinen Formel

R^{I}-O-H

darstellen. R stellt dabei einen verzweigten oder unverzweigten Alkylrest einer Kettenlänge von C₁₋₂₅ dar. R^{I} stellt einen verzweigten oder unverzweigten C₁₋₂₅-Alkylrest dar, wobei auch ein oder mehrere Wasserstoffatome dieses Alkylrestes durch eine oder mehrere OR^{II}- Reste substituiert sein können, wobei dieser R^{II}-Rest oder diese - R^{II}-Reste unabhängig voneinander aus der Gruppe H, verzweigtes oder unverzweigtes C₁₋₂₅-Alkyl und/oder verzweigtes oder unverzweigtes C₁₋₂₅-Acyl gewählt werden können.

Vorteilhaft können diese Reste so gewählt werden, daß R dabei einen verzweigten oder unverzweigten Alkylrest einer Kettenlänge von C₆₋₁₈ darstellt, R^{I} aus der Gruppe verzweigtes oder unverzweigtes C₁₋₁₀-Alkyl-, wobei auch ein oder mehrere Wasserstoffatome dieses Alkylrests durch eine oder mehrere OR^{II}- Reste substituiert sein können, wobei dieser R^{II}-Rest oder diese -R^{II}-Reste unabhängig voneinander aus der Gruppe H, verzweigtes oder unverzweigtes C₆₋₁₈-Alkyl oder C₆₋₁₈-Acyl gewählt werden können.

Besonders vorteilhaft stellt R dabei einen Alkylrest einer Kettenlänge von C₆₋₁₈ dar, R^{I} wird günstig gewählt aus der Gruppe
Hexyl-, Isopropyl-, sowie aus der Gruppe 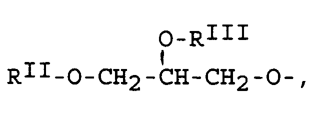
wobei R^{II} und R^{III} unabhängig voneinander aus der Gruppe H-, verzweigtes oder unverzweigtes C₆₋₁₈-Alkyl- oder C₆₋₁₈-Acylgewählt werden können.

Bevorzugt ist insbesondere, die Fettsäureester zu wählen aus der Gruppe Hexyllaurat, Isopropylstearat, Glycerylmonolaurat, Caprylsäure-/Caprinsäuretriglycerid.

Die erfindungsgemäßen Verbindungen werden vorzugsweise in dermatologischen Zusammensetzungen eingesetzt, insbesondere entsprechend einem Gehalt von 0,01 - 50,0 Gew.-%, bevorzugt 0,01 - 20,0 Gew.-%, bezogen auf das Gesamtgewicht der Zusammensetzung. Vorteilhaft enthalten die Zusammensetzungen 0,02 - 10,0 Gew.-%, besonders bevorzugt 0,02 - 5,0 Gew.-% an den erfindungsgemäßen ethoxylierten bzw. propoxylierten organischen Verbindungen, ganz besonders vorteilhaft 0,5 - 3,0 Gew.-%, jeweils bezogen auf das Gesamtgewicht der Zusammensetzung.

Die erfindungsgemäßen Verbindungen lassen sich ohne Schwierigkeiten in gängige dermatologische Formulierungen einarbeiten, vorteilhaft in Pumpsprays, Aerosolsprays, Crèmes, Salben, Tinkturen, Lotionen und dergleichen.

Es ist auch möglich und gegebenenfalls vorteilhaft, die erfindungsgemäßen Verbindungen mit anderen Wirkstoffen zu kombinieren, beispielsweise mit antimikrobiell wirksamen Stoffen.

Zusätzlich zu den genannten Bestandteilen können den erfindungsgemäßen dermatologischen Zubereitungen Parfüm, Farbstoffe, Antioxidantien, Suspendiermittel, Puffergemische oder andere übliche kosmetische oder dermatologische Grundstoffe beigemischt werden.

Erfindungsgemäß können als günstige Antioxidantien alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-; Amyl-, Butyl- und Lauryl-, Palmitoyl-, Oleyl-, γ-Linoleyl-, Cholesteryl - und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall)-Chelatoren (z.B.α-Hydroxyfettsäuren, Palmitinsäure, Phytinsäure, Lactoferrin), α-Hydroxysäuren (z.B. Zitronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin A-palmitat) sowie Koniferylbenzoat des Benzoeharzes, Rutinsäure und deren Derivate, Ferulasäure und deren Derivate, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO₄) Selen und dessen Derivate (z.B. Selenmethionin), Stilbene und deren Derivate (z.B. Stilbenoxid, TransStilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Es ist vorteilhaft, die erfindungsgemäßen Zusammensetzungen abzupuffern. Vorteilhaft ist ein pH-Bereich von 3,5 - 7,5. Besonders günstig ist es, den pH-Wert in einem Bereich von 4,0 - 6,0 zu wählen.

Die erfindungsgemäßen dermatologischen Formulierungen können wie üblich zusammengesetzt sein und zur Behandlung der Haut im Sinne einer dermatologischen Behandlung dienen.

Zur Anwendung werden die erfindungsgemäßen Formulierungen in der für Dermatika üblichen Weise auf die Haut in ausreichender Menge aufgebracht.

Die erfindungsgemäßen dermatologischen Zubereitungen können Hilfsstoffe enthalten, wie sie üblicherweise in solchen Zubereitungen verwendet werden, z.B. Konservierungsmittel, Bakterizide, Parfüme, Mittel zum Verhindern des Schäumens, Farbstoffe, Pigmente, die eine färbende Wirkung haben, Verdickungsmittel, oberflächenaktive Substanzen, Emulgatoren, weichmachende Substanzen, anfeuchtende bzw. feuchthaltende Substanzen, Fette, Öle, Wachse oder andere übliche Bestandteile einer dermatologischen Formulierung wie Alkohole, Polyole, Polymere, Schaumstabilisatoren, Elektrolyte, organische Lösungsmittel oder Silikonderivate.

Sofern die dermatologische Zubereitung eine Lösung oder Lotion darstellt, können als Lösungsmittel verwendet werden:
- Wasser oder wäßrige Lösungen;
- Öle, wie Triglyceride der Caprin- oder der Caprylsäure, vorzugsweise aber Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte

Insbesondere werden Gemische der vorstehend genannten Lösungsmittel verwendet. Bei alkoholischen Lösungsmitteln kann Wasser ein weiterer Bestandteil sein.

Gele gemäß der Erfindung enthalten üblicherweise Alkohole niedriger C-Zahl, z.B. Ethanol, Isopropanol, 1,2-Propandiol, Glycerin und Wasser bzw. ein vorstehend genanntes Öl in Gegenwart eines Verdickungsmittels, das bei ölig-alkoholischen Gelen vorzugsweise Siliciumdioxid oder ein Aluminiumsilikat wie beispielsweise Bentonite, bei wäßrigalkoholischen oder alkoholischen Gelen vorzugweise ein Polyacrylat ist. Weitere Beispiele für vorteilhafte Verdickungsmittel sind Gummiarabikum, Xanthangummi, Natriumalginat, Cellulose-Derivate, vorzugsweise Methylcellulose, Hydroxymethylcellulose, Hydroxyethylcellulose, Hydroxypropylcellulose, Hydroxypropylmethylcellulose oder anorganische Verdickungsmittel oder ein Gemisch aus Polyethylenglykol und Polyethylenglycolstearat oder -distearat. Das oder die Verdickungsmittel sind in einem Gel z.B. in einer Menge zwischen 0,1 und 30 Gew.-%, bevorzugt zwischen 0,5 und 15 Gew.-%, enthalten.

Feste Stifte gemäß der Erfindung enthalten z.B. natürliche oder synthetische Wachse, Fettalkohole oder Fettsäureester.

Als Treibmittel für erfindungsgemäße, aus Aerosolbehältern versprühbare dermatologische Zubereitungen sind die üblichen bekannten leichtflüchtigen, verflüssigten Treibmittel, beispielsweise Kohlenwasserstoffe (Propan, Butan, Isobutan) geeignet, die allein oder in Mischung miteinander eingesetzt werden können. Auch Druckluft ist vorteilhaft zu verwenden.

Natürlich weiß der Fachmann, daß es an sich nichttoxische Treibgase gibt, die grundsätzlich für die vorliegende Erfindung geeignet wären, auf die aber dennoch wegen bedenklicher Wirkung auf die Umwelt oder sonstiger Begleitumstände verzichtet werden sollte, insbesondere Fluorkohlenwasserstoffe und Fluorchlorkohlenwasserstoffe (FCKW).
Bevorzugt können die erfindungsgemäßen Zubereitungen außerdem Substanzen enthalten, die UV-Strahlung absorbieren, wobei die Gesamtmenge der Filtersubstanzen z.B. 0,1 Gew.-% bis 30 Gew.-%, vorzugsweise 0,5 bis 10 Gew.-%, insbesondere 1 bis 6 Gew.-% beträgt, bezogen auf das Gesamtgewicht der Zubereitung.

Die nachfolgenden Beispiele sollen die vorliegende Erfindung verdeutlichen, ohne sie einzuschränken.

### Beispiel 1

| Sonnenschutzcrème | |
|---|---|
| | Gew.-% |
| Glycerylcocoat + hydriertes Kokosöl + Ceteareth-25 ("Softisan 601") | 35,00 |
| Caprylsäure-/Caprinsäuretriglycerid ("Miglyol 812") | 7,00 |
| Glycerylstearat ("Imwitor 960") | 5,00 |
| Caprylsäurediethylamid ("Repellent 790") | 3,00 |
| Dihydroxyaceton | 0,50 |
| Phenylbenzimidazolsulfonsäure ("Eusolex 232") | 4,00 |
| Konservierungsmittel, Parfum, Stabilisatoren usw. | nach Belieben |
| Wasser | ad 100,00 |

### Beispiel 2

| Tagescrème | |
|---|---|
| | Gew.-% |
| Glycerylstearat ("Imwitor") | 15,00 |
| Caprylsäure/Caprinsäure-Triglycerid ("Miglyol 812") | 15,00 |
| Paraffinöl DAB 9 | 5,00 |
| Glycerin-Polyethylenglycolricinoleat ("Cremophor EL") | 2,00 |
| Konservierungsmittel, Parfum, Stabilisatoren usw. | nach Belieben |
| Wasser | ad 100,00 |

### Beispiel 3

| Kollagencrème | |
|---|---|
| | Gew.-% |
| Glycerylstearat ("Imwitor") | 9,00 |
| Paraffinöl DAB 9 | 5,00 |
| Isopropylmyristat | 5,00 |
| Cetylalcohol | 1,00 |
| Stearinsäure | 5,00 |
| Caprylsäure/Caprinsäure-Triglycerid ("Miglyol 812") | |
| | 5,00 |
| Sorbitlösung 70 % | 5,00 |
| Triethanolamin | 0,90 |
| Kollagen CLR | 5,00 |
| Konservierungsmittel, Parfum, Stabilisatoren usw. | nach Belieben |
| Wasser | ad 100,00 |

### Beispiel 4

| O/W-Diglycerincrème | |
|---|---|
| Phase A | Gew.-% |
| Glycerylstearat ("Imwitor 900") | 9,50 |
| Cetearylalkohol ("Lanette O" | 1,60 |
| Cetearylisethionat ("Cetiol SN") | 4,00 |
| Cetylacetat + Acetylierter Lanolinalkohol ("Acetulan") | 2,40 |
| Calendulaöl | 1,60 |
| Isopropylstearat | 0,75 |
| Glycerinmonolaurat | 0,75 |

| Phase B | |
|---|---|
| Diglycerin | 8,00 |
| Glycerin | 8,00 |
| Carbomer/Polyacrylat ("Carbopol 940") | 0,12 |
| Triethanolamin 98 % | 0,22 |
| Kamillenextrakt | 1,00 |
| Konservierungsmittel, Parfum, Stabilisatoren usw. | nach Belieben |
| Wasser | ad 100,00 |

Die Phasen A und B werden separat auf 60° C erwärmt, unter Rühren zusammengegeben und sodann langsam auf Raumtemperatur abgekühlt.

### Beispiel 5

| Ölbad | |
|---|---|
| | Gew.-% |
| Oleth-5 ("Eumulgin O 5") | 10,00 |
| Laurinsäurehexylester ("Cetiol A") | 40,00 |
| Cocosfettsäurediethanolamid ("Comperlan") | 10,00 |
| Caprylsäure-/Caprinsäure-Triglycerid ("Myritol 318") | 10,00 |
| Paraffinöl DAB 9 | 25,00 |
| Parfum | 5,00 |

### Beispiel 6

| Ölbad | |
|---|---|
| | Gew.-% |
| Fettalkoholpolyether ("Aethoxal B") | 52,00 |
| Laureth-2 ("Dehydol LS 2" | 10,00 |
| Laurinsäurehexylester ("Cetiol A") | 15,00 |
| Caprylsäure-/Caprinsäure-Triglycerid ("Myritol 318") | 20,00 |

## Patentansprüche

1. Verwendung eines oder mehrerer Fettsäureester ein- und/oder mehrwertiger Alkohole als Wirkprinzip gegen Superinfektionen.

2. Verwendung nach Anspruch 1, dadurch gekennzeichnet, daß die Fettsäurestern gewählt werden aus der Gruppe der Verbindung der allgemeinen Formel 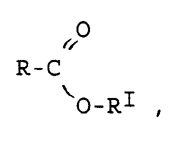 wobei
R einen verzweigten oder unverzweigten Alkylrest einer Kettenlänge von C₁₋₂₅ darstellt,
R^{I} einen verzweigten oder unverzweigten C₁₋₂₅-Alkylrest darstellt, wobei auch ein oder mehrere Wasserstoffatome dieses Alkylrestes durch eine oder mehrere OR^{II}- Reste substituiert sein können, wobei dieser R^{II}-Rest oder diese -R^{II}-Reste unabhängig voneinander aus der Gruppe H, verzweigtes oder unverzweigtes C₁₋₂₅-Alkyl und/oder verzweigtes oder unverzweigtes C₁₋₂₅-Acyl gewählt werden.

3. Verwendung nach Anspruch 2, dadurch gekennzeichnet, daß R einen verzweigten oder unverzweigten Alkylrest einer Kettenlänge von C₆₋₁₈ darstellt, R^{I} aus der Gruppe verzweigtes oder unverzweigtes C₁₋₁₀-Alkyl-, wobei auch ein oder mehrere Wasserstoffatome dieses Alkylrests durch eine oder mehrere OR^{II}- Reste substituiert sein können, wobei dieser R^{II}-Rest oder diese -R^{II}-Reste unabhängig voneinander aus der Gruppe H, verzweigtes oder-unverzweigtes C₆₋₁₈-Alkyl oder C₆₋₁₈-Acyl gewählt werden.

4. Verwendung nach Anspruch 2, dadurch gekennzeichnet, daß R einen Alkylrest einer Kettenlänge von C₆₋₁₈ darstellt, R^{I} gewählt wird aus der Gruppe Hexyl-, Isopropyl-, sowie aus der Gruppe 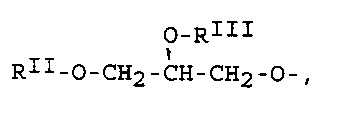 wobei R^{II} und R^{III} unabhängig voneinander aus der Gruppe H-, verzweigtes oder unverzweigtes C₆₋₁₈-Alkyl- oder C₆₋₁₈-Acylgewählt werden.

5. Verwendung nach Anspruch 1, dadurch gekennzeichnet, daß die Fettsäureester gewählt werden aus der Gruppe Hexyllaurat, Isopropylstearat, Glycerylmonolaurat, Caprylsäure/Caprinsäuretriglycerid.

6. Verwendung nach Anspruch 1, dadurch gekennzeichnet, daß die Fettsäureester ein- und/oder mehrwertiger Alkohole in kosmetischen oder dermatologischen Zusammensetzungen in einem Gehalt von 0,01 - 20,0 Gew.-%, bevorzugt in einem Gehalt von 0,02 - 5,0 Gew.-% besonders bevorzugt in einem Gehalt von 0,5 - 3,0 Gew.-% vorliegen, jeweils bezogen auf das Gesamtgewicht der Zusammensetzung.
